## Europäisches Patentamt

## European Patent Office

## Office européen des brevets

(11) Publication number: **0 216 538**
A1

# EUROPEAN PATENT APPLICATION

(21) Application number: 86306747.6

(51) Int. Cl.⁴: **A 61 B 17/06**

(22) Date of filing: 02.09.86

(30) Priority: 06.09.85 GB 8522182

(43) Date of publication of application:
01.04.87 Bulletin 87/14

(84) Designated Contracting States:
CH DE FR IT LI NL SE

(71) Applicant: **MICRA Ltd.**
54 Alma Street
Luton Bedfordshire LU1 2PL(GB)

(72) Inventor: **Hoskin, William John**
5, Long Buftlers
Harpenden Hertfordshire AL5 1JF(GB)

(72) Inventor: **Pierse, Dermot John**
143, Harly Street
London W1N 1DJ(GB)

(74) Representative: **Griffin, Kenneth David  et al,**
Saunders & Dolleymore 2 Norfolk Road
Rickmansworth Hertfordshire WD3 1JH(GB)

(54) Surgical needle.

(57) A needle of the kind for use in surgery, especially microsurgery, for stitching human tissue with a suture is characterised by a body portion (1) and a tip (2) which is secured to the body portion. The tip (2) is of a material which is harder than that of the body portion (1) and has a sharp cutting edge. The tip material may be a gem such as a diamond, sapphire or ruby, or an amorphous compound of titatium, cobalt and chromium having the properties of glass rather than metal which is sold under the registered trade mark "METGLAS".

FIG.1

EP 0 216 538 A1

-1-

SURGICAL NEEDLE

This invention relates to a needle (hereinafter referred to as "a needle of the kind hereinbefore specified") for use in surgery, especially micro-surgery, for stitching human tissue with a suture.

Needles of the kind hereinbefore specified are presently manufactured from a piece of tempered stainless steel wire which is placed in a machine which by an impact from a die on a round anvil causes a U-shaped section to be formed on the wire out of the material itself. This U-shaped section is then closed over by a drawing or swaging process with a mandrel in place which results in a circular bore being produced at one end of this very fine wire. This method is used for inserting sutures into the ends of very fine needles.

For many surgical operations such needles do not have sufficiently sharp cutting edges so that damage to the tissue being stitched may result.

It is an object of the present invention to provide a surgical needle which enables the danger of such damage to be substantially reduced or eliminated.

According to the present invention a needle of the kind hereinbefore specified is characterised in that it comprises a body portion and a tip which is secured to the body portion, the tip being of a material which is harder than that of the body portion and which has a sharp cutting edge.

The material of the tip is preferably a gem, such as a diamond, sapphire or ruby.

Another example of a said material is an amorphous material being a compound of titanium, cobalt and chromium which, however, has substantially

-2-

the properties of glass rather than metal and which is sold under the registered trade mark "METGLAS".

Preferably the tip has a shank portion and the tip is secured to the body portion of the needle by insertion of said shank portion in a bore in the end of the body portion opposite the end into which the suture is inserted as aforesaid, the said bore being made in the same way as the aforesaid bore for the suture.

Preferably, however, the tip is secured to the body portion by brazing or gluing it to the latter by its aforesaid shank portion.

Preferably, the tip is pre-sharpened before it is secured to the body portion and will need no further treatment.

Tips for use in needles according to the invention can be manufactured on machinery presently being used to manufacture styli for record players. Such styli are made in both diamond and sapphire and with some minor modification to the machine and to the shape of the end, the machine could be made suitable for manufacture the said tips.

A needle embodying the invention should be sufficiently inexpensive for it to be sold in a pack already sterilised as a disposable needle with a suture attached.

Using modern laser techniques it is possible to drill the end of a said body portion of a needle, e.g. with the aid of a so-called "neodymium YAG laser". Such a procedure is proposed instead of deforming the material of the body portion by a mechanical process as aforesaid.

The body portion of the needle is of course formed into the shape required before the tip is inserted, so as to avoid the danger of damage to the tip during this forming step.

Various forms of needle embodying the invention will now be described, by way of example only, with reference to the accompanying drawings in which:-

Figure 1 shows a first said form of needle complete with a suture;

Figure 2 is a side view of the tip for insertion into the body portion of the needle of Figure 1;

Figures 3A, 3B and 3C are respectively a side view, end view and partly sectioned side view of the body portion of the needle of Figure 1;

Figures 4A, 4B and 4C are perspective views of respectively a blank for a said body portion and two subsequent stages in the manufacture of the body portion by mechanical deformation;

Figure 5 shows a re-usable said needle complete with a handle therefor; and

Figures 6A and 6B are fragmentary views of the body portion ends and tips of two forms of needle of the kind shown in Figure 5.

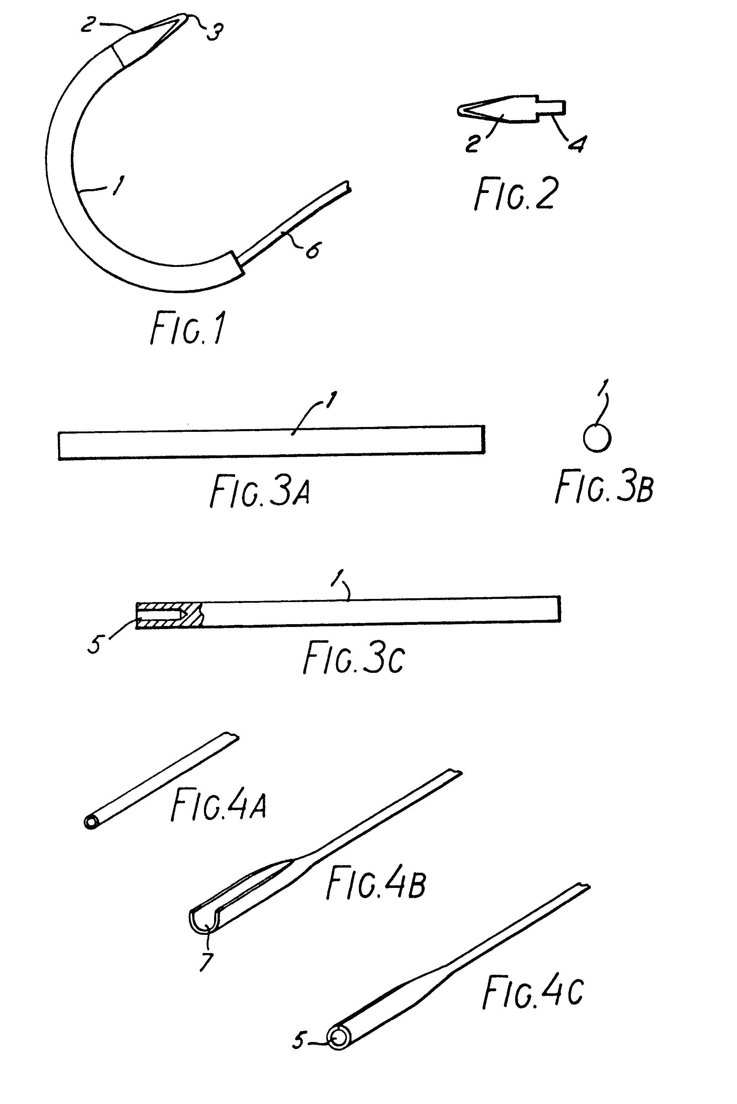

Referring to Figure 1, the needle comprises a body portion 1 and a tip 2 which is secured to the body portion 1 in a manner to be described hereinafter with reference to Figures 2 to 4. The body portion 1 is of tempered stainless steel wire and the material of the tip 2, which has a sharp cutting edge 3, is harder than that of the body portion 1 viz. a gem such as ruby, sapphire or diamond.

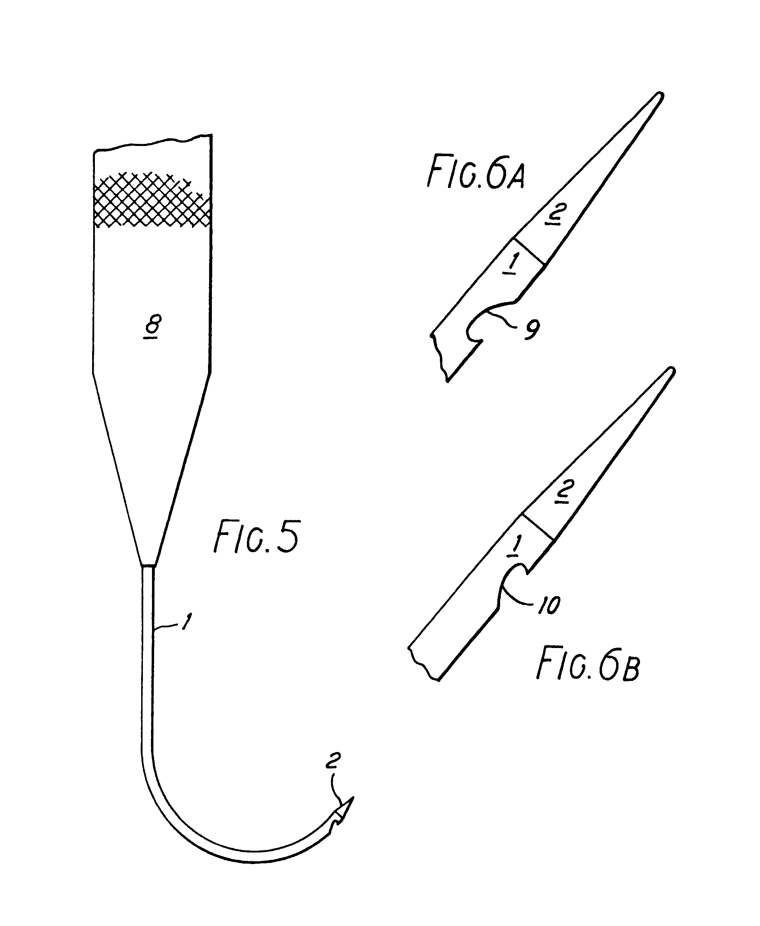

The tip 2 is shown in greater detail in Figure 2, including its shaft 4 which is cylindrical and inserted into a bore 5 (Figures 3C and 4C) in one end of the body portion 1, and brazed or glued therein.

A bore similar to the bore 5 is produced in the other end of the body portion 1 and one end of a suture 6 (Figure 1) is secured in that bore by the state of the art process hereinbefore mentioned.

Figures 3A and 3B show the blank body portion 1 prior to the production therein of the bores for the tip 2 and the suture 6. In the body portion 1 shown in Figures 3A-C, the bore 5 (and, if desired, the bore for the suture 6) is/are produced by a laser drill.

In the alternative method of producing the bore 5 (and, if desired, the bore for the suture 6) the blank body portion 1 shown in Figure 4A is, as hereinbefore mentioned, placed in a machine which by an impact from a die on a round anvil causes the formation of the U-shaped section 7 (Figure 4B) which is then closed over by a drawing or swaging process with a mandrel in place which results in the circular bore 5 being produced (Figure 4C).

In the re-usable form of needle shown in Figure 5, the body portion 1 is secured to a handle 8 which may or may not be a permanent part of the needle.

The body portion 1 of the needle (which is made in precisely the same manner as that described with reference to Figures 1 to 4) has a notch 9 (Figure 6A) or 10 (Figure 6B) cut into it and, when the needle is inserted in the human tissue (e.g. of an eye) it either carries a loop of suture with it (with the notch shaped as shown in Figure 6A) or it hooks around the piece of suture and pulls the latter back when the needle is withdrawn from the tissue (for which the purpose the notch is as shown in Figure 6B). Hence the direction in which the

-5-

notch 9, 10 is placed determines which of these two last-mentioned procedures is used. The notch 9, 10 is cut by a laser, and this method of suturing has been found to work extremely well.

At the present time surgeons have a preference for using continuous sutures and disposable needles. On the other hand it is now being shown that interrupted suturing (viz. employing a needle as shown in Figures 5 and 6) for certain purposes could be of material benefit.

The main uses of needles of the kind hereinbefore described are for ophthalmology, plastic surgery, joining of tendons and suturing walls of blood vessels which are extremely muscular and hard. The ophthalmic needle is the smallest that would be required, but the present invention is not restricted to its application to ophthalmology; indeed the benefits of the present invention may turn out to be of greatest significance in its application to some or all of the other fields of surgery mentioned.

CLAIMS

1.      A needle for stitching tissue with a suture characterised in that it comprises a body portion (1.) and a tip (2) which is secured to the body portion, the tip being of a material which is harder than that of the body portion and which has a sharp cutting edge.

2.      A needle according to Claim 1 characterised in that the material of the tip is a gem.

3.      A needle according to Claim 2 characterised in that the gem is a diamond.

4.      A needle according to Claim 2 characterised in that the gem is a sapphire.

5.      A needle according to Claim 2 characterised in that the gem is a ruby.

6.      A needle according to Claim 1 characterised in that the material of the tip is an amorphous compound of titanium, cobalt and chromium having substantially the properties of glass.

7.      A needle according to any one of the preceding claims characterised in that the tip (2) has a shaft (4), and that the tip is secured to the body portion (1) of the needle by insertion of said shaft in a first bore (5) in a first end of the body portion opposite a second bore in a second end of the body portion into which the suture (6) is inserted, said first bore (5) being made in the same way as said second bore.

8.      A needle according to any one of the preceding claims characterised in that the tip (2) is secured to the body portion (1) by brazing or gluing.

9.      A needle according to any one of the preceding claims characterised in that the tip (2) is pre-sharpened before it is secured to the body portion (1).

10.     A needle according to any one of the preceding claims characterised in that it is in combination with a handle (8) to define a re-usable needle structure.

0216538

1/2

FIG.1

FIG.2

FIG.3A

FIG.3B

FIG.3c

FIG.4A

FIG.4B

FIG.4C

FIG.5

FIG.6A

FIG.6B

**0216538**

European Patent
Office

**EUROPEAN SEARCH REPORT**

Application number

EP  86 30 6747

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | EP-A-0 119 714  (HPW LTD.) <br> * Abstract; page 1, lines 1-21 * <br><br> --- | 1 | A 61 B   17/06 |
| A | CH-A-  621 476  (LABORATOIRE SUISSE DE RECHERCHES HORLOGERES NEUCHATEL) <br> * Abstract;  claims 1,2; figures 4,5 * <br><br> --- | 1 | |
| P,A | EP-A-0 167 330  (G.A. KIM) <br> * Abstract; page 7, lines  25-29; figure 5 * <br><br> ----- | 1 | |

TECHNICAL FIELDS
SEARCHED (Int Cl 4)

A 61 B
B 42 B
B 21 G
A 61 F
D 05 B
B 26 F

The present search report has been drawn up for all claims

| Place of search | Date of completion of the search | Examiner |
|---|---|---|
| THE HAGUE | 02-12-1986 | NEILL M.C. |